# NOUVEAU FASCICULE DE BREVET EUROPEEN

(11) **EP 2 519 179 B2**
(45) Date de publication et mention de la décision concernant l'opposition: **02.12.2020**
(45) Mention de la délivrance du brevet: 07.02.2018
(21) Numéro de dépôt: 10801595.9
(22) Date de dépôt: 17.11.2010
(51) Int. Cl.: A61B 17/88

(54) **EQUIPEMENT POUR LA POSE D'UN IMPLANT**
VORRICHTUNG ZUR POSITIONIERUNG EINES IMPLANTATS
DEVICE FOR POSITIONING AN IMPLANT

(30) Priorité: 28.12.2009 FR 0906369
(43) Date de publication de la demande: 07.11.2012
(73) Titulaire: Safe Orthopaedics, 95610 Eragny-sur-Oise (FR)
(72) Inventeur: PETIT, Dominique, F-62180 Verton (FR)
(74) Mandataire: IP Trust
(86) Numéro de dépôt international: PCT/FR2010/000772
(87) Numéro de publication internationale: WO 2011/080404

(56) Documents cités:
- EP-A2- 1 295 578
- WO-A1-2005/016183
- WO-A1-2005/016183
- WO-A2-2008/097974
- DE-C1- 4 007 306
- US-A- 4 257 129
- US-A- 5 716 415
- US-A1- 2001 032 017
- US-A1- 2005 027 360
- US-A1- 2005 038 431
- US-A1- 2005 165 408
- US-A1- 2006 004 376
- US-A1- 2006 074 445
- US-A1- 2006 276 803
- US-A1- 2007 016 221
- US-A1- 2008 262 318
- US-A1- 2009 222 045
- US-B1- 6 174 334

## Description

L'invention concerne un équipement pour la pose d'un implant.

L'équipement est destiné notamment, mais non exclusivement, à la pose d'une cage inter somatique, d'un clou fémoral, d'une vis pédiculaire rachidienne, d'une prothèse discale ou d'une plaque d'ostéosynthèse rachidienne.

De manière classique en soi, les équipements utilisés pour la pose d'implant comportent une partie filetée apte à coopérer avec une partie taraudée ménagée dans l'implant. Les demandes de brevet WO96/22747 et FR2668360 décrivent de tels équipements. Ainsi, le montage de l'implant sur l'équipement s'effectue en général en engageant par vissage la partie filetée dans l'alésage taraudé de l'implant. Une fois l'implant positionné dans l'emplacement prévu, l'équipement de pose est libéré de l'implant en dévissant la partie filetée de l'alésage taraudé.

De tels équipements ne permettent pas cependant un montage et démontage rapide de l'implant du fait des opérations de vissage et dévissage.

Un second inconvénient est que le blocage au moyen d'une partie filetée impose, une fois l'implant posé, le démontage de l'équipement afin de poursuivre l'intervention chirurgicale.

On connait également des équipements de pose d'implants comprenant un corps tubulaire pourvu de deux bras présentant des extrémités d'accroche de l'implant, comme décrit dans le document US 2006/276803. Dans ce type d'équipement, le blocage de l'implant sur les bras est assuré au moyen d'un tube de blocage externe, ledit tube étant positionné sur l'équipement par déplacement le long de la face externe du corps tubulaire.

Ce type d'équipement présente cependant plusieurs inconvénients.

Un premier inconvénient est que le système de blocage mis en oeuvre n'est pas adapté aux équipements de pose à usage unique. Les bras des équipements à usage unique sont en effet plus fragiles de par la nature du matériau dans lequel l'équipement est réalisé. Or, le blocage de l'implant par la mise en place d'une pièce de blocage autour des bras accentue cette fragilité.

Un deuxième inconvénient est que, outre la fragilité qu'il confère à l'équipement, la présence d'une pièce externe au corps tubulaire de l'équipement conduit à un équipement relativement encombrant.

Un autre inconvénient est que le blocage au moyen d'une pièce externe n'est adapté qu'à un instrument de forme droite. Il ne peut être mis en oeuvre avec des équipements de forme complexe, ce qui limite le champ d'application.

L'invention vise à remédier aux problèmes de l'art antérieur en proposant un équipement de pose d'implant peu invasif permettant une pose d'implant précise ainsi qu'une prise et une libération d'implant rapide.

L'invention a également pour but de proposer un équipement de pose permettant, en complément de l'étape de pose de l'implant, la réalisation d'étapes complémentaires intervenant au cours de l'intervention chirurgicale.

L'invention a également pour but de proposer un équipement de pose résistant et d'encombrement optimum, offrant un maintien fiable de l'implant et répondant à la miniaturisation des implants et des instruments chirurgicaux.

A cet effet, et selon un premier aspect, l'invention propose un équipement pour la pose d'un implant comprenant un instrument de préhension de l'implant comprenant deux portions aptes à prendre une position de maintien de l'implant et une position ouverte autorisant la préhension ou la libération de l'implant, et un élément de blocage des portions en position de maintien, l'élément de blocage comprenant des moyens d'accroche agencés pour venir en prise avec des moyens -d'accroche complémentaires ménagés dans un logement de réception formé par les portions en position de maintien de l'implant, lesdits moyens formant une liaison glissière.

Ainsi, l'équipement obtenu lorsque l'instrument de préhension et l'élément de blocage sont assemblés offre une résistance mécanique améliorée en flexion et torsion et assure une bonne fixation de l'implant sur l'instrument. Par ailleurs le contrôle des portions de l'instrument assurant la préhension de l'implant ou sa libération au moyen d'un élément interne permet de limiter l'encombrement.

Avantageusement, la liaison glissière est configurée pour permettre un mouvement de translation rectiligne de l'élément de blocage par rapport à l'instrument de préhension. Selon une variante de réalisation, il peut être prévu une liaison glissière configurée pour permettre un mouvement de translation circulaire de l'élément de blocage par rapport à l'instrument de préhension.

Selon une configuration avantageuse, chacune des extrémités proximales est agencée pour venir en prise avec l'implant.

Il peut être prévu également que l'implant soit pré-monté sur les extrémités proximales des portions.

Selon un mode de réalisation avantageux, les moyens d'accroche de l'élément de blocage comprennent des saillies en forme de queue d'aronde aptes à s'insérer dans des rainures de forme complémentaire ménagées dans le logement, et inversement. Cela permet ainsi d'offrir une liaison glissière bilatérale. Par ailleurs, l'équipement obtenu lorsque l'instrument de préhension et l'élément de blocage sont assemblés offre une résistance mécanique améliorée en flexion et torsion et assure la tenue de l'implant.

Avantageusement, les portions sont formées par deux demi-coquilles assemblées l'une avec l'autre par une pièce de solidarisation.

Selon une configuration particulière, l'élément de blocage et la pièce de solidarisation sont formés d'un seul tenant.

Il peut être prévu également que les deux demi-coquilles soient jointes l'une à l'autre par une bague de maintien positionnée en extrémité distale desdites demi-coquilles.

Selon un mode de réalisation particulier, l'équipement comporte un dispositif de visée comprenant au moins un orifice permettant la visée et la pose d'un organe de fixation en association avec l'implant une fois celui posé. Celui-ci peut être porté soit par l'élément de blocage, soit par l'instrument de préhension.

Avantageusement, l'équipement comporte au moins un deuxième implant pré-monté sur l'élément de blocage. Selon une configuration particulière, le deuxième implant est porté par le dispositif de visée.

Avantageusement, l'instrument de préhension est un tube de montage.

Avantageusement, l'instrument de préhension, lorsque l'élément de blocage (4) est positionné en son sein, forme un tube de guidage.

Avantageusement, l'équipement est réalisé en matière composite, polymère, alliage ferreux ou non ferreux ou encore une combinaison de ses différentes matières. Le matériau utilisé, outre sa biocompatibilité, sera avantageusement recyclable.

Avantageusement, l'équipement est à usage unique.

Avantageusement, l'équipement peut être mis en oeuvre avec un implant fémoral, un implant rachidien du type cage inter somatique, vis pédiculaire rachidienne, prothèse discale, ou plaque d'ostéosynthèse rachidienne. Il est bien entendu évident que l'équipement ne se limite pas à de tels implants.

D'autres objets et avantages de l'invention apparaîtront au cours de la description qui suit, faite en référence aux dessins annexés, dans lesquels :
- la figure 1 représente une vue schématique d'un équipement pour la pose d'une cage inter somatique selon l'invention, la cage étant montée sur l'équipement ;
- la figure 2 représente une vue de l'équipement de la figure 1 avant l'insertion de l'élément de blocage à l'intérieur de l'instrument de préhension ;
- la figure 3 représente une vue de l'équipement de la figure 1, l'élément de blocage étant partiellement inséré à l'intérieur de l'instrument de préhension ;
- la figure 3a représente une vue en coupe selon l'axe III-III de l'équipement de la figure 3 ;
- les figures 4 à 6 représentent respectivement une vue de détail de l'équipement illustré respectivement en figure 2, 3 et 1 ;
- la figure 7 représente une vue schématique d'un équipement pour la pose d'un clou fémoral selon l'invention ;
- la figure 8 représente une vue de détail en coupe selon l'axe I-I de l'équipement de la figure 7 ;
- les figures 9 à 12 représentent des vues montrant les étapes de montage de l'équipement de la figure 7, le clou fémoral étant fixé à l'équipement ;
- la figure 13 représente l'équipement de la figure 7 équipé d'un dispositif de visée et d'un guide pour permettre le positionnement d'une vis trochantérique ;
- la figure 14 représente l'équipement de la figure 7 équipé d'un dispositif de visée et d'un guide pour permettre le positionnement d'une vis cervicale ;
- la figure 15 représente une vue partielle éclatée d'un équipement pour la pose d'une plaque d'ostéosynthèse selon l'invention, la plaque étant montée sur l'équipement ;
- la figure 16 représente une vue de l'équipement de la figure 15, avant l'insertion de l'élément de blocage à l'intérieur de l'instrument de préhension ;
- la figure 17 représente une plaque d'ostéosynthèse destinée à être associée à l'équipement de pose de la figure 15 ;
- les figures 18 et 19 représentent respectivement la plaque d'ostéosynthèse en cours de montage sur l'instrument de préhension et la plaque une fois montée ;
- les figures 20 et 21 représentent une vue en perspective respectivement de côté et de dessus de l'équipement de la figure 15, le dispositif de visée de l'élément de blocage étant représenté lors de son utilisation en association avec un taraud ;
- la figure 22 représente une vue en perspective de dessus de l'équipement de la figure 15, le dispositif de visée étant représenté muni de vis pré montées ;
- la figure 23 représente une vue en coupe du dispositif de visée selon l'axe II-II de la figure 22 ;
- les figures 24 et 25 représentent une vue en perspective respectivement de côté et de dessus d'un instrument de préhension d'une plaque d'ostéosynthèse selon un mode de réalisation ;
- la figure 26 représente l'instrument de préhension des figures 24 et 25 pourvu d'un dispositif de visée ;
- la figure 27 représente une vue en perspective de dessus de l'instrument de préhension de la figure 26, le dispositif de visée étant représenté lors de son utilisation en association avec un taraud ; et
- la figure 28 représente une vue en perspective de l'instrument de préhension de la figure 26, le dispositif de visée étant représenté équipé de vis pré-montées.

Pour plus de clarté, les éléments identiques ou similaires des différents modes de réalisation sont repérés par des signes de référence identiques sur l'ensemble des figures.

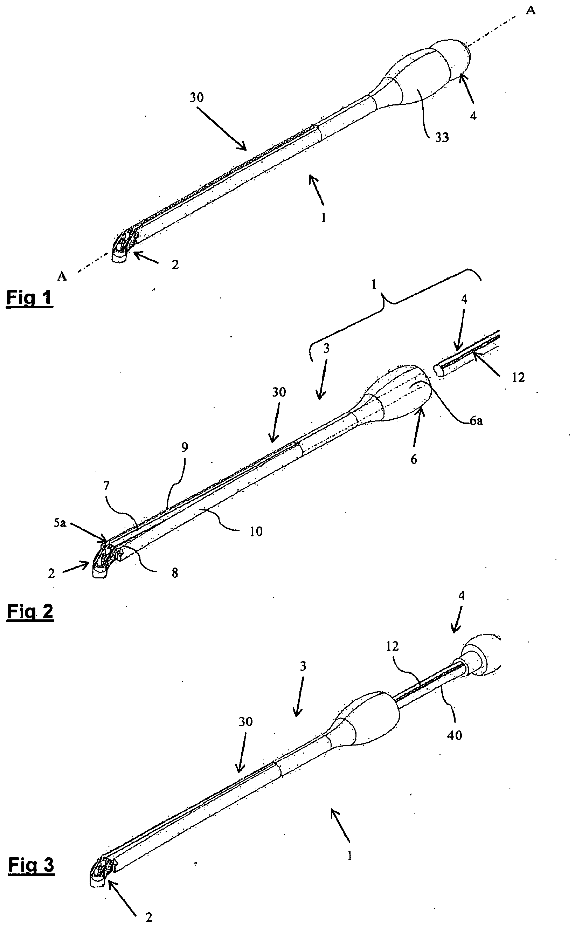

En relation avec les figures 1 à 6, il est décrit un équipement 1 pour la pose d'une cage inter somatique 2 entre deux vertèbres rachidiennes.

La forme de la cage mise en oeuvre avec l'équipement selon l'invention n'est pas limitée à la forme illustrée sur les figures 1 à 6. En effet, la forme d'une cage varie selon les abords par lesquels l'intervention chirurgicale s'effectue (abords antérieur, postérieur ou latéral) et selon le type de région de la colonne vertébrale dans laquelle la cage est implantée (régions cervicale, thoracique ou lombaire). Cependant, et comme on le verra plus loin, la cage destinée à être associée à l'équipement comprend des moyens spécifiques permettant sa prise par l'équipement en question.

L'équipement 1 pour la pose d'une cage inter somatique 2 comprend un premier élément formant un instrument de préhension 3 de la cage 2 et un deuxième élément formant un élément de blocage 4 assurant le maintien de la cage 2 par l'instrument lorsque celle-ci est en prise par l'instrument. Les moyens mis en oeuvre pour permettre un tel maintien seront décrit en détail plus loin.

Dans le mode de réalisation décrit, l'instrument de préhension 3 comporte un tube de montage 30 présentant une extrémité distale 6 ouverte pour permettre le passage de l'élément de blocage 4 et une extrémité proximale 5 agencée pour permettre la préhension de la cage 2.. Par « extrémité proximale », on entend l'extrémité la plus proche de l'implant lorsqu'il est associé à l'instrument de préhension 3, et par « extrémité distale », on entend l'extrémité la plus éloignée de l'implant lorsqu'il est associé à l'instrument de préhension 3.

Comme on le verra plus loin, le tube de montage 30 est réalisé en forme et dimension pour pouvoir recevoir en son sein l'élément de blocage 4.

Avantageusement, le tube de montage 30 comporte deux fentes longitudinales 7, 8 s'étendant depuis l'extrémité proximale 5 en direction de l'extrémité distale 6 de manière à délimiter deux portions 9, 10 longitudinales formant bras de préhension. Avantageusement, les fentes longitudinales 7, 8 sont agencées de manière à former deux bras de préhension symétriques rapport à l'axe AA du tube de montage 30.

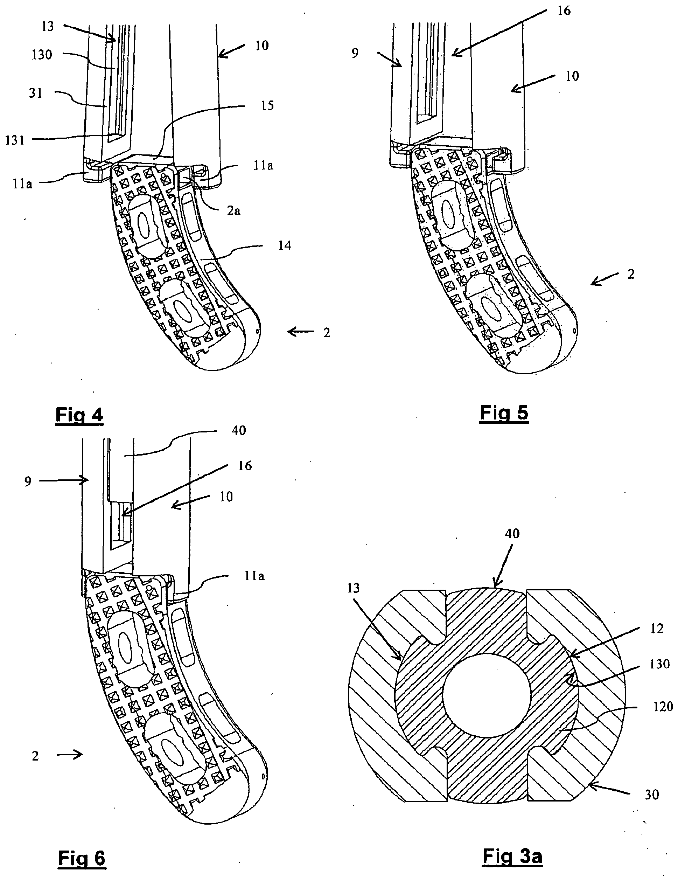

Afin de faciliter la préhension de la cage 2 mais également sa libération une fois implantée entre les deux vertèbres, les fentes 7, 8 sont dimensionnées de manière à ce que les portions 9, 10 formant bras présentent une certaine flexibilité. Par flexibilité, on entend la capacité des portions 9, 10 formant bras de préhension de s'écarter dans une direction opposée à l'axe AA du tube de montage 30 et de se rapprocher en direction de ce même axe. C'est de part cette flexibilité que les portions 9, 10 sont aptes à prendre une position de maintien de l'implant comme illustrée sur la figure 6 et une position ouverte autorisant la préhension ou la libération de la cage comme illustrée sur la figure 4.

Avantageusement, l'extrémité proximale 5 du tube de montage 30 est agencée pour venir en prise avec la cage 2 (figures 4 à 6). Plus particulièrement, chacun des bras 9, 10 comporte, au niveau de leur extrémité définissant l'extrémité proximale 5 du tube de montage, un ergot 11a s'étendant vers l'intérieur du tube de montage 30. Les ergots 11a sont agencés pour venir se loger respectivement dans un évidement 2a de forme complémentaire prévu à cet effet dans la cage 2. Dans le mode de réalisation décrit, les évidements 2a sont ménagés sur les faces latérales 14 de la cage, à proximité de la face arrière 15. On définit le terme « arrière » par rapport à la position des faces de la cage lors de son insertion entre les vertèbres. Ainsi, la face arrière correspondant à la face opposée à la face avant par laquelle la cage 2 est implantée. Il est bien entendu évident que l'invention ne se limite pas, en ce qui concerne les moyens de maintien de l'implant au tube de montage, aux ergots tels qu'illustrés sur les figures 4 à 6, toutes autres formes d'ergots ou tout autre moyen de maintien d'implant pouvant être prévus sans pour autant sortir du cadre de l'invention.

Comme on le comprend des figures 4 à 6, la préhension de la cage 2 est réalisée par rapprochement des portions 9, 10 l'une vers l'autre. Ce rapprochement s'effectue par insertion d'une portion tubulaire 40 de l'élément de blocage 4 à l'intérieur du tube de montage 30, au travers de l'ouverture de passage 6a d'extrémité distale 6 vers l'extrémité proximale 5, comme illustré sur la figure 3 et ce, jusqu'à sa mise en butée sur la face 131 du tube de montage 3. Une fois la portion tubulaire 40 insérée, la cage 2 est fermement maintenue par les portions 9, 10 du tube de montage 30. Elle ne dispose alors plus d'aucun degré de liberté de mouvement. Il est à noter qu'entre la position ouverte dans laquelle les portions 9, 10 sont écartées l'une de l'autre (figure 4) et la position rapprochée dans laquelle les portions 9, 10 maintiennent fermement la cage (figure 6), il existe une position intermédiaire (figure 5), dans laquelle les portions 9, 10 sont suffisamment resserrées pour empêcher la désolidarisation de la cage des portions 9, 10 mais suffisamment écartées l'une de l'autre pour permettre, le cas échéant, un ajustement de la position la cage 2 par rapport au tube de montage 30. Cet agencement présente un intérêt tout particulier lorsque les moyens de maintien de l'implant sont de forme circulaire, la position intermédiaire autorisant alors un ajustement de la position angulaire.

Afin d'assurer le rapprochement des portions 9, 10 de l'instrument de préhension 3 tout en assurant une connexion rigide entre la cage inter somatique 2 et l'instrument de préhension 3, l'élément de blocage 4 comporte avantageusement des moyens d'accroche 12 agencés pour venir en prise avec des moyens d'accroche complémentaires 13 ménagés dans un logement de réception 16 de l'élément de blocage 4 formé par les portions en position de maintien de l'implant, lesdits moyens formant une liaison glissière.

Dans un mode de réalisation préféré, les moyens d'accroche 12, ménagés sur la portion tubulaire 40 de l'élément de blocage 4 et les moyens d'accroche de forme complémentaire 13 ménagés à l'intérieur du tube de montage 30, sur la face interne 31 délimitant le logement de réception 16, présentent un profil du type queue d'aronde, comme illustré sur la figure 3a. La complémentarité des formes de la portion tubulaire 40 de l'élément de blocage 4 et des portions 9, 10 du tube de montage 30 a pour avantage de rigidifier l'équipement lorsque l'élément de blocage est placé à l'intérieur du tube de montage et ainsi d'améliorer sa résistance.

Ainsi, dans le mode de réalisation décrit, les moyens d'accroche 12 portés par l'élément de blocage 4 comprennent des saillies longitudinales 120 en forme de trapèze (queue d'aronde contenue), les moyens d'accroche complémentaires 13 portés par le tube de montage 30 étant formés par une rainure 130 de forme complémentaire à celle des saillies (queue d'aronde contenante). Il est bien entendu évident qu'il peut être prévu un équipement avec des moyens d'accroche inversés (queue d'aronde contenue portée par le tube de montage 30 et queue d'aronde contenante portée par l'élément de blocage 4). Avantageusement, les saillies sont agencées pour former une queue d'aronde continue s'étendant longitudinalement entre l'extrémité proximale 5 et l'extrémité proximale 6 du tube de montage 30.

Dans les modes de réalisation précédemment décrits, la liaison glissière est rectiligne (ie. elle est configurée de manière à permettre un mouvement de translation rectiligne de l'élément de blocage 4 à l'intérieur de l'instrument de préhension 3). Il est bien entendu évident que l'invention ne se limite pas à la forme de l'instrument de préhension 3 décrit et donc à ce type de liaison. En particulier, il peut être prévu des moyens d'assemblage de l'élément de blocage avec l'instrument de préhension formant une liaison glissière circulaire.

Afin de faciliter la manipulation de la cage 2 et assurer une meilleure préhension de l'instrument 3, le tube de montage 30 présente, en partie distale une forme bombée 33 configurée pour assurer une fonction de poignée.

Comme on le verra plus loin, il pourra être avantageux, en prévision d'une livraison conjointe de la cage 2 et de l'équipement de pose 1 de prévoir que la cage 2 soit pré-montée sur les extrémités proximales 5, 6 des portions 9, 10.

Selon un mode de réalisation préférentiel, l'équipement est à usage unique.

Avantageusement, l'équipement de pose 1 est réalisé en matériau composite, polymère, alliage ferreux ou non ferreux ou encore une combinaison de ces différentes matériaux.

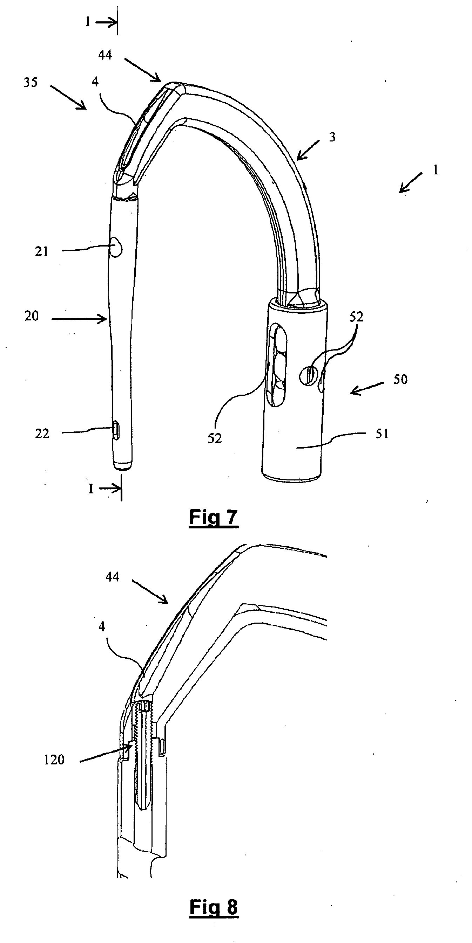

En relation avec les figures 7 à 14, il est décrit un équipement 1 pour la pose d'un clou fémoral 20 et de vis de fixation associées (non représentées) en vue de la consolidation d'un fémur suite à une rupture du col associé. Dans cet exemple de réalisation, l'instrument de préhension reprend pour l'essentiel l'ensemble des caractéristiques de l'instrument de préhension mis en oeuvre avec la cage inter somatique 2. La description de ces caractéristiques ne sera donc pas reprise dans son intégralité. Seules les caractéristiques spécifiques de l'équipement liées à son utilisation avec un clou fémoral seront donc décrites.

Par vis de fixation associées, on entend d'une part les vis trochantériques utilisées pour l'ancrage du clou fémoral 20 au niveau de la diaphyse et d'autre part la vis cervicale utilisée pour l'ancrage céphalique. Le clou fémoral 20 quant à lui est une tige tubulaire, de forme générale cylindrique légèrement cintrée.

Comme dans l'exemple précédemment décrit, l'équipement de pose 1 comprend un instrument de préhension 3 du clou fémoral 20 et un élément de blocage 4 permettant le maintien du clou fémoral 20 par l'instrument de préhension ou bien sa libération une fois le clou fémoral implanté dans l'emplacement prévu.

L'instrument de préhension 3 se présente sous la forme d'un corps tubulaire assimilable au tube de montage 30 précédemment décrit.

Le corps ou tube de montage 30 comporte, en partie distale 32, une poignée 33 permettant la manipulation manuelle de l'instrument 3. La poignée 33, sensiblement droite, est prolongée par une partie allongée 34 courbe dont l'extrémité est conformée en une tête 35 incurvée. La pointe de la tête 35 définit l'extrémité proximale 5 du tube de montage 30.

Dans le mode de réalisation décrit, le tube de montage 30 est formé à partir de deux demi-coquilles 30a, 30b de forme et de dimensions identiques. Les deux demi-coquilles 30a, 30b sont disposées en regard l'une de l'autre et jointes l'une à l'autre au niveau de leurs bords longitudinaux. Avantageusement, les deux demi-coquilles 30a, 30b sont agencées pour permettre une liaison réversible. Dans le mode de réalisation décrit, la liaison s'effectue par clipsage des parties 33a, 33b des deux demi-coquilles formant la poignée 33. Comme on le comprendra plus loin, cet assemblage au niveau de la poignée 33 est un assemblage primaire, les deux demi-coquilles 30a, 30b étant par la suite reliées entre elles au moyen d'une pièce de solidarisation distincte 44. Cette pièce de solidarisation 44 sera décrite plus loin. Il est bien entendu évident que les deux demi-coquilles 30a, 30b peuvent être assemblées l'une à l'autre par tout autre moyen, comme par exemple par clipsage ou emboîtage ou par l'intermédiaire d'un manchon de maintien (non représenté). Par ailleurs, l'assemblage primaire peut ne pas être prévu sans pour autant sortir du cadre de l'invention.

Avantageusement, les deux demi-coquilles 30a, 30b sont agencées de manière à ce que, lorsqu'elles sont jointes pour former le tube de montage 30, une fente 36 débouchant à l'intérieur du tube de montage 30 et s'étendant en partie sur la tête 35 et la partie courbe 34 allongée est formée. Cette fente 36 constitue une ouverture permettant le passage de la pièce de solidarisation 44 et son positionnement à l'intérieur du tube de montage 30, entre les deux demi-coquilles 30a, 30b.

La pièce de solidarisation 44 est formée et dimensionnée de manière à s'emboîter dans le logement de réception délimité par la face interne des deux demi-coquilles 30a, 30b et la fente 36, formant ainsi une portion rigide.

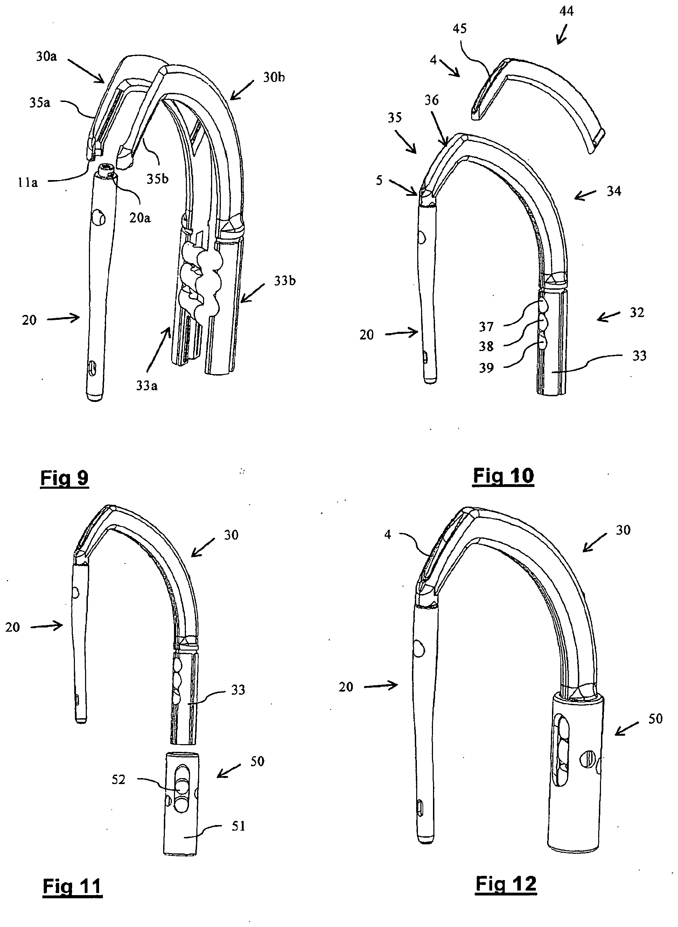

Dans ce mode de réalisation, l'élément de blocage 4 permettant d'empêcher tout écartement des portions 35a, 35b de la tête 35 est formé d'un seul tenant avec la pièce de solidarisation 44 (figure 10). A cet effet, la pièce de solidarisation 44 et la partie 35a, 35b formant tête 35 comporte des moyens de blocage de type queue d'aronde similaires à ceux décrit précédemment.

Comme on le comprend des figures 9 et 10, la préhension du clou fémoral 20 est réalisée en présentant les deux demi-coquilles 30a, 30b disjointes l'une de l'autre. La préhension du clou fémoral 20 est réalisée par rapprochement des deux demi-coquilles 30a, 30b l'une vers l'autre jusqu'à venir se positionner l'une contre l'autre. A ce moment là, l'extrémité proximale de chacune des deux demi-coquilles définissant l'extrémité proximale 5 du tube de montage 30 enserre l'extrémité haute du clou. On définit le terme « haut» par rapport à la position du clou fémoral 20 lorsqu'il est implanté dans le fémur. En d'autres termes, l'extrémité haute correspond à l'extrémité la plus proche du col du fémur.

Les deux demi-coquilles 30a, 30b forment alors le tube de montage 30. Une fois le clou fémoral 20 maintenu entre les extrémités proximale des deux demi-coquilles 30a, 30b, la pièce de solidarisation 44 est placée dans le logement de réception 16 par mise en contact et glissement des queues d'aronde contenues (ou contenantes) ménagés sur la portion de tête 45 de la pièce de solidarisation 44 dans les queues d'aronde complémentaires ménagées sur la face interne de chacune des deux demi-coquilles 30a, 30b, au niveau des parties 35a, 35b formant tête 35, en direction de l'ouverture d'extrémité proximale 5. Une fois la pièce de solidarisation 44 entièrement insérée entre les deux demi-coquilles 30a, 30b, l'écartement des parties 35a, 35b des deux demi-coquilles formant la tête est empêché et la fixation du clou fémoral 20 sur l'instrument de préhension 3 assuré.

Comme dans l'exemple précédemment décrit, l'extrémité proximale 5 de la tête 35 est agencée pour venir en prise avec le clou fémoral 20. Ainsi, les parties 35a, 35b formant tête, assimilables aux portions 9, 10 de l'équipement précédemment décrit, comportent respectivement un ergot 11a s'étendant vers l'intérieur du tube de montage 30 et agencés pour venir se loger respectivement dans un évidement 20a, de forme complémentaire prévu à cet effet dans le clou fémoral 20. Dans le mode de réalisation décrit, les évidements 20a sont ménagés au niveau de l'extrémité haute 25 du clou fémoral 20.

Selon une configuration de réalisation particulièrement avantageuse, l'instrument de préhension 3 est ménagé de manière à pouvoir porter au moins un deuxième implant. La figure 8 illustre à cet effet la pièce de solidarisation 44 pourvue, au niveau de l'extrémité de la portion de tête 45, d'une vis de pression 120 destinée à venir bloquer par friction la vis trochantérique. La vis de pression 120 est fixée par vissage dans une ouverture d'extrémité taraudée de la portion de tête 45 de la pièce de solidarisation 44. La vis de pression 120 ainsi préalablement chargée sur la pièce de solidarisation 44, et plus précisément sur l'élément de blocage 4, prévient ainsi toute désolidarisation et renforce la connexion. Cela évite également tout démontage préalable à la fin de la mise en place de l'élément de blocage. Un tel pré-chargement d'implant permet par ailleurs de réduire le temps d'intervention chirurgicale.

Avantageusement un tel pré-chargement d'implant permet un geste chirurgical de précision. Il n'est pas toujours aisé lors d'une chirurgie de solidariser deux implants entre eux. L'équipement 1 dans son ensemble répond à cette problématique. Dans cet exemple bien précis d'une application sur un clou fémoral 20, l'instrument de préhension 3 et la pièce de solidarisation 44 forme un guide pour la mise en place de la vis de pression 120.

Avantageusement, la poignée 33 est creuse (figure 9). Afin de permettre le passage de guides pour la mise en place des vis trochantériques et/ou cervicale, des trous de passage 37, 38, 39 transversaux, parallèles entre eux, sont prévus sur la poignée 33. Chaque trou, en association avec l'un des alésages 21, 22 ménagés dans le clou fémoral 20, définit une position angulaire déterminé pour les vis trochantériques ou cervicale. Dans le mode de réalisation décrit, la poignée 33 est représentée avec trois trous de passage 37, 38, 39 transversaux correspondant à trois positions angulaires privilégiées. Il est bien entendu évident que la poignée 33 peut être pourvue d'un nombre différent de trous de passage.

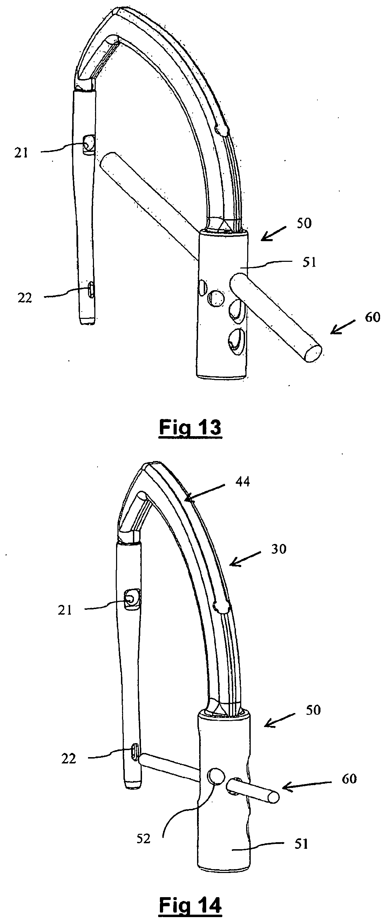

Selon une variante de réalisation, l'instrument de préhension 3 comporte un dispositif de visée 50 permettant la visée des vis trochantériques et cervicale. Dans le mode de réalisation décrit, le dispositif de visée 50 comporte une bague de visée 51 apte à s'emboiter sur la poignée 33. La bague de visée 51 est dimensionnée de manière à assurer sa tenue sur la poignée 33 tout en autorisant un mouvement de rotation sur celle-ci. Avantageusement, la bague de visée 51 comprend plusieurs lumières 52 agencées pour permettre, selon la position donnée à la bague de visée 51 par rapport à la poignée 33, des positionnements angulaires spécifiques des vis trochanthériques et cervicale. Ainsi, les figures 13 et 14 illustrent la bague de visée 38 traversée par un guide 60 pour le passage des vis de fixation, la bague de visée 51 étant en position de visée pour la vis trochantérique sur la figure 13 et en position de visée pour la vis cervicale (figure 14). La présence d'une bague de visée 51 permet ainsi de proposer un nombre de positions angulaires supérieur aux trois positions angulaires classiquement prévues au niveau des poignées.

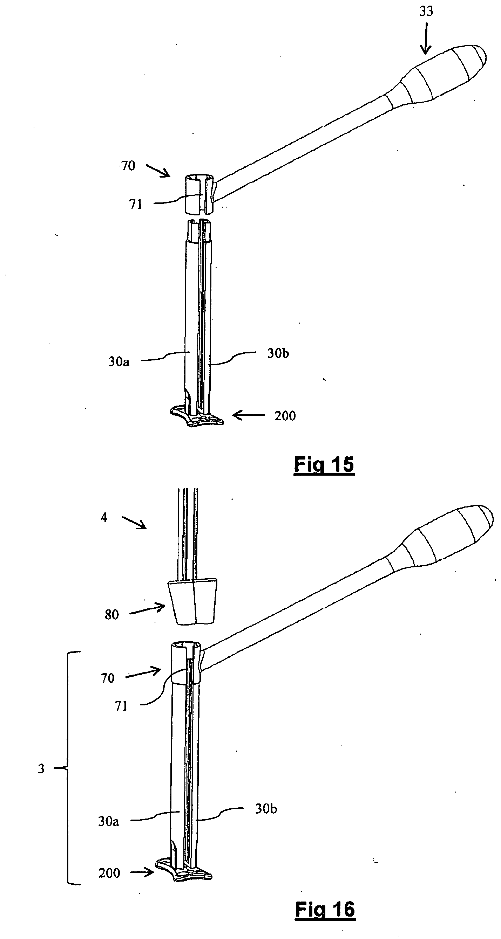

Les figures 15 à 23 illustrent un équipement 1 mis en oeuvre pour la pose d'une plaque d'ostéosynthèse rachidienne 200. Comme précédemment, l'équipement 1 comprend un instrument de préhension 3 de la plaque 200 et un élément de blocage 4 assurant le maintien de la plaque 200 par l'instrument de préhension 3 lorsque celle-ci est en prise par l'instrument. Dans l'exemple de réalisation décrit ci-après, l'instrument de préhension 3 est une combinaison des caractéristiques de l'instrument de préhension mis en oeuvre avec la cage inter somatique 2 et de l'instrument de préhension mis en oeuvre aven le clou fémoral. La description de ces caractéristiques ne sera donc pas reprise dans son intégralité. Seules les caractéristiques spécifiques de l'équipement liées à son utilisation avec la plaque d'ostéosynthèse seront décrites.

La forme de la plaque mise en oeuvre avec l'équipement n'est pas limitée à la forme illustrée sur les figures 15 à 23, celle-ci pouvant varier selon les abords par lesquels les interventions chirurgicales s'effectuent (abords antérieur, postérieur ou latéral) et selon le type de régions de la colonne vertébrale dans laquelle la plaque est destinée (régions cervicale, thoracique ou lombaire) ainsi que pour toute autre chirurgie orthopédique générale traumatologique.

Dans cet exemple de réalisation, comme pour le clou fémoral, l'instrument de préhension 3 est un tube de montage 30 formé de deux demi-coquilles 30a, 30b. Cependant, dans le cas présent, les deux demi-coquilles sont maintenues à distance et en regard l'une de l'autre et sont reliées l'une à l'autre au moyen d'une bague de maintien 70 positionnée en extrémité distale des deux demi-coquilles 30a, 30b. Ainsi formé, l'instrument de préhension 3 est assimilable au tube de montage 30 mis en oeuvre avec la cage inter somatique 2. Ainsi, les deux demi-coquilles correspondent respectivement à une des deux portions 9, 10 formant bras de préhension et l'espacement présent entre les deux demi-coquilles correspond au logement de réception 16 et aux fentes 7, 8. Comme on le verra plus loin, l'espacement présent entre les deux demi-coquilles va permettre le passage d'un élément de blocage muni d'un dispositif de visée.

Avantageusement, la bague de maintien 70 comporte une fente 'étendant sur toute sa longueur. Lors de la mise en place de la bague de maintien 70 au niveau des extrémités des deux demi-coquilles, il sera pris soin de positionner la fente 71 dans le prolongement de l'espacement présent entre les deux demi-coquilles pour permettre le passage d'un élément de blocage muni d'un dispositif de visée.

Selon une variante de réalisation, l'instrument de préhension pourrait être formé à partir d'un corps tubulaire droit dans lequel a été réalisé une fente s'étendant depuis l'extrémité d'ouverture proximale jusqu'à l'extrémité d'ouverture distale.

La bague de maintien 70 permet de maintenir provisoirement les deux demi coquilles 30a, 30b en position assemblée jusqu'à la mise en place de l'élément de blocage 4 qui prendra alors le relais une fois inséré à l'intérieur des deux demi-coquilles 30a, 30b. La bague de maintien 70 forme ainsi un moyen de maintien primaire.

L'instrument de préhension 3 comporte une poignée 33 agencée pour faciliter la manipulation du tube de montage 30. Dans un mode de réalisation avantageux, la poignée 33 et la bague de maintien 70 sont solidaires l'une de l'autre.

Comme cela a été décrit précédemment, la fonction première de l'élément de blocage 4 est la solidarisation des demi-coquilles 30a, 30b à l'aide de la liaison glissière du type queue d'aronde.

Dans le mode de réalisation décrit, l'élément de blocage 4 comporte, au niveau de l'une de ses extrémités, un dispositif de visée 80 permettant de guider la pose d'un élément d'ancrage osseux, tel qu'une vis de fixation 90, pour la fixation de la plaque d'ostéosynthèse 200. Avantageusement, l'élément de blocage 4 et le dispositif de visée 80 forme une seule et même pièce.

Le dispositif de visée 80 comprend des orifices de visée 81, chaque orifice étant ménagé de sorte à présenter un axe de visée colinéaire avec une des lumières 201 ménagées dans la plaque 200 et destinée à recevoir une vis de fixation 90. Dans le mode de réalisation illustré, le dispositif de visée 80 comprend deux orifices de visée 81 d'axes de visée colinéaires.

Avantageusement, le dispositif de visée est solidaire de l'élément de blocage par une portion de jonction 82 configurée pour coulisser le long de la fente 71 de la bague de maintien 70 et entre les deux demi-coquilles 30a, 30b.

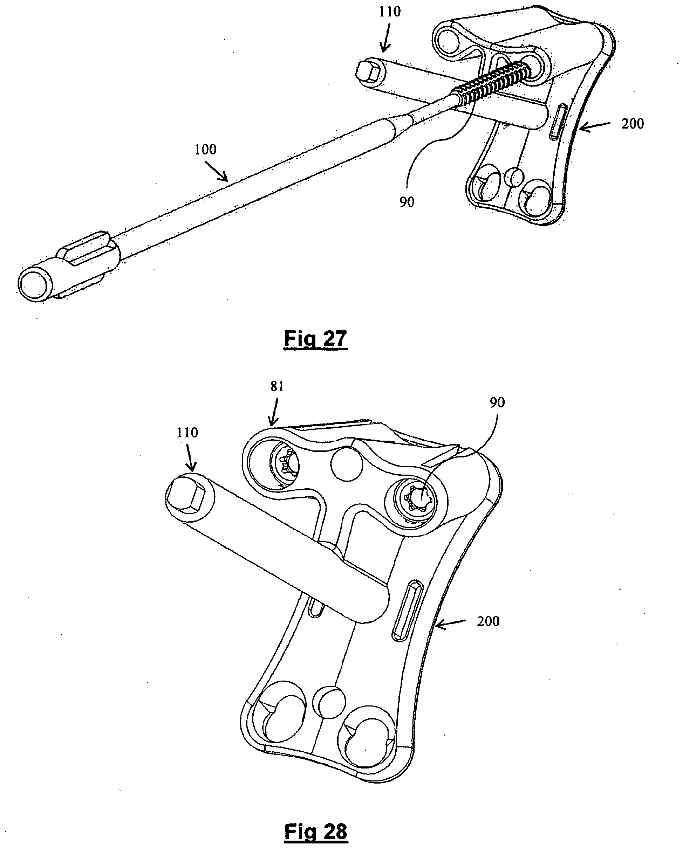

Avantageusement, les orifices de visée 81 sont agencés pour recevoir une vis de fixation 90 mais également pour permettre le passage de tarauds 100 ou de tout autre type d'instrument tels que mèches, sondes , tournevis, etc.(figure 27).

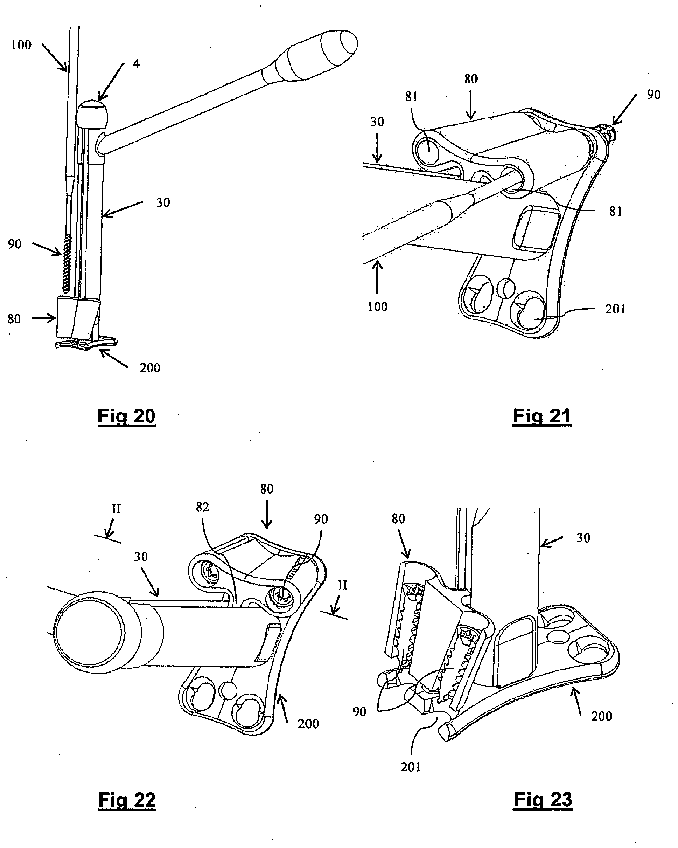

Dans un mode de réalisation particulièrement avantageux, illustré par les figures 22 et 23, il est prévu un élément de blocage 4 comprenant un dispositif de visée dans lequel les vis de fixation 90 sont pré-montées dans les orifices de visée 81. Le dispositif de visée forme alors un « distributeur de vis».

La présence conjointe d'un dispositif de visée et de vis de fixation pré-montées permet au chirurgien d'avoir un geste précis, rapide et simplifié.

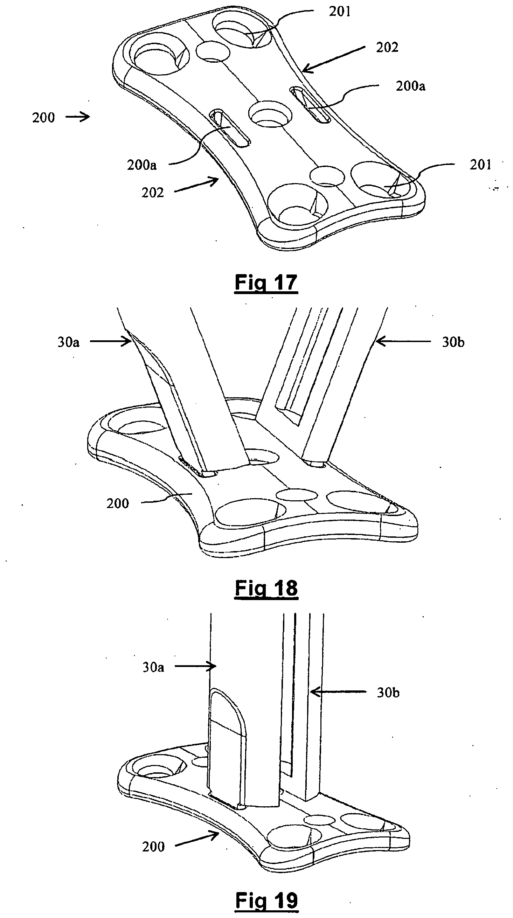

Comme dans l'exemple précédemment décrit, l'extrémité proximale 5 du tube de montage 30 formée par les extrémités proximales des deux demi-coquilles sont agencés pour venir en prise avec la plaque 200. Ainsi, l'extrémité proximale de chacune des demi-coquilles 30a 30b comportent respectivement un ergot s'étendant vers l'intérieur du tube de montage et agencé pour venir se loger respectivement dans un évidement, de forme complémentaire prévu à cet effet dans la plaque 200. Dans le mode de réalisation décrit, les évidements sont ménagés au niveau des bords latéraux 202 de la plaque 200 (figures 17 à 19). On entend par « bords latéraux », les bords s'étendant sensiblement dans la même direction que la direction longitudinale de la plaque.

Les figures 18 et 19 illustrent le montage de la plaque sur l'instrument de préhension. Il est à noter, et on le comprend plus aisément avec l'exemple de la plaque, que la réalisation d'un instrument de préhension au moyen de deux demi-coquilles distinctes a pour avantage de faciliter et d'améliorer la préhension de l'implant.

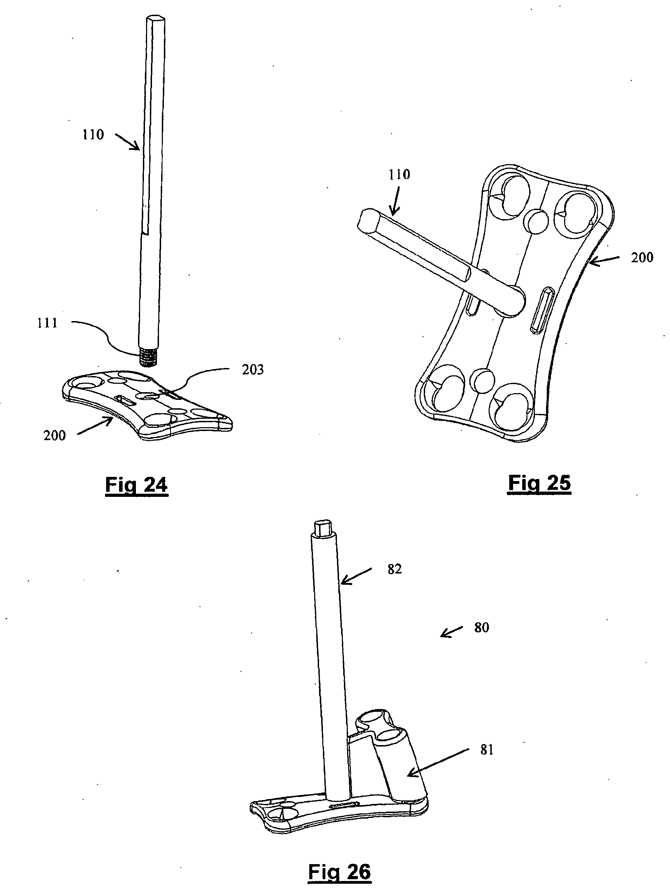

Les figures 24 à 28 illustrent une version simplifiée de l'équipement 1 de pose d'une plaque d'ostéosynthèse, l'équipement étant pourvu d'un dispositif de visée 80 tel que décrit précédemment. L'instrument de préhension 3 reprend donc l'ensemble des caractéristiques de l'instrument de préhension de pose d'une plaque précédemment décrit. Seules les caractéristiques spécifiques de cet équipement sont donc décrites ci-après.

Dans ce mode de réalisation, l'équipement 1 de pose comprend une tige épaulée 110 présentant une extrémité filetée 111 destinée à coopérer avec un orifice taraudé 203 prévu à cet effet dans la plaque 200. Dans le mode de réalisation décrit, l'orifice taraudé 203 est positionné au centre de la plaque 200.

Avantageusement, le dispositif de visée 80 comprend un corps tubulaire 82 configuré pour s'emboiter sur la tige épaulée 110.

En vue des orientations actuelles du « prêt à l'emploi, l'équipement de pose selon l'invention et l'implant associé, une fois assemblés, sont avantageusement disposés dans un emballage stérile pour être ensuite livrés aux hôpitaux. Il sera également avantageux de prévoir un équipement de pose à usage unique. La préparation d'un tel conditionnement a pour avantage de réduire le coût global de la chirurgie et de garantir la non contamination d'un patient à un autre. Cela a également pour avantage de diminuer le temps d'intervention chirurgicale, et par conséquent de procéder à des anesthésies du patient plus courtes.

## Revendications

1. Equipement (1) pour la pose d'un implant (2, 20, 200) comprenant :
- un instrument de préhension (3) de l'implant (2, 20, 200) comprenant deux portions (9, 10, 30a, 30b) aptes à prendre une position de maintien de l'implant (2, 20, 200) et une position ouverte autorisant la préhension ou la libération de l'implant (2, 20, 200) une fois celui-ci implanté, et
- un élément de blocage (4) des portions (9, 10, 30a, 30b) en position de maintien de l'implant, l'élément de blocage comprenant des moyens d'accroche (13, 130) agencés pour venir en prise avec des moyens d'accroche complémentaires (12) ménagés dans un logement de réception (16) formé par les portions (9, 10, 30a, 30b) en position de maintien de l'implant, lesdits moyens formant une liaison glissière.

2. Equipement (1) selon la revendication 1, **caractérisé en ce que** la liaison glissière est configurée pour permettre un mouvement de translation rectiligne de l'élément de blocage (4) par rapport à l'instrument de préhension (3).

3. Equipement (1) selon la revendication 1 ou la revendication 2, **caractérisé en ce que** la liaison glissière est configurée pour permettre un mouvement de translation circulaire de l'élément de blocage (4) par rapport à l'instrument de préhension (3).

4. Equipement (1) selon l'une quelconque des revendications 1 à 3, **caractérisé en ce que** les portions (9, 10, 30a, 30b) présentent chacune une extrémité proximale agencée pour venir en prise avec l'implant (2, 20, 200).

5. Equipement (1) selon la revendication 4, caractérisé en que l'implant (2, 20, 200) est pré-monté sur les extrémités proximales des portions (9, 10, 30a, 30b).

6. Equipement (1) selon l'une quelconque des revendications 1 à 5, **caractérisé en ce que** les moyens d'accroche (12) de l'élément de blocage (4) comprennent des saillies en forme de queue d'aronde aptes à s'insérer dans des rainures (130) de forme complémentaire ménagées dans le logement (16), et inversement.

7. Equipement (1) selon l'une quelconque des revendications 1 à 6, **caractérisé en ce que** les portions sont formées par deux demi-coquilles (30a, 30b) assemblées l'une à l'autre par une pièce de solidarisation (44).

8. Equipement (1) selon la revendication 7, **caractérisé en ce que** l'élément de blocage (4) et la pièce de solidarisation (44) sont formés d'un seul tenant.

9. Equipement (1) selon la revendication 7 ou la revendication 8, **caractérisé en ce que** les deux demi-coquilles (30a, 30b) sont jointes l'une à l'autre par une bague de maintien (70) positionnée en extrémité distale desdites demi-coquilles.

10. Equipement (1) selon l'une quelconque des revendications 1 à 9, caractérisé en qu'il comporte un dispositif de visée (50, 80) comprenant au moins un orifice (52, 81) permettant la visée et la pose d'un organe de fixation en association avec l'implant une fois celui posé.

11. Equipement (1) selon la revendication 10, **caractérisé en ce que** le dispositif de visée (50, 80) est porté par l'élément de blocage (4).

12. Equipement (1) selon la revendication 10, **caractérisé en ce que** le dispositif de visée (50, 80) est porté par l'instrument de préhension (3).

13. Equipement (1) selon l'une quelconque des revendications 1 à 12, **caractérisé en ce qu'**il comporte au moins un deuxième implant (90, 120) pré-monté sur l'élément de blocage (4).

14. Equipement (1) selon l'une quelconque des revendications 11 à 13, **caractérisé en ce que** le deuxième implant (90, 120) est porté par le dispositif de visée (50, 80).

15. Equipement (1) selon l'une quelconque des revendications 1 à 14, **caractérisé en ce que** l'instrument de préhension (3) est un tube de montage (30).

16. Equipement (1) selon l'une quelconque des revendications 1 à 15, l'instrument de préhension (3), lorsque l'élément de blocage (4) est positionné en son sein, forme un tube de guidage.

17. Equipement (1) selon l'une quelconque des revendications 1 à 16, **caractérisé en ce que** l'équipement (1) est réalisé en matière composite, polymère, alliage ferreux ou non ferreux ou encore une combinaison de ses différentes matières.

18. Equipement (1) selon l'une quelconque des revendications 1 à 17, **caractérisé en ce que** l'équipement (1) est à usage unique.

19. Equipement (1) selon l'une quelconque des revendications 1 à 18, **caractérisé en ce que** l'implant (2, 20, 200) est un implant fémoral (20).

20. Equipement (1) selon l'une quelconque des revendications 1 à 18, **caractérisé en ce que** l'implant (2, 20, 200) est un implant rachidien (2, 200) du type cage inter somatique ou plaque d'ostéosynthèse rachidienne.

## Patentansprüche

1. Vorrichtung (1) für das Einsetzen eines Implantats (2, 20, 200), welche Folgendes umfasst:
- ein Instrument zum Greifen (3) des Implantats (2, 20, 200), welches zwei Abschnitte (9, 10, 30a, 30b) umfasst, die in der Lage sind, eine Halteposition zum Greifen des Implantats (2, 20, 200) und eine offene Position zum Loslassen des Implantats (2, 20, 200) nach dessen Implantation einzunehmen, und
- ein Element zum Arretieren (4) der Abschnitte (9, 10, 30a, 30b) in der Halteposition des Implantats, wobei das Arretierelement Befestigungsmittel (13, 130) umfasst, die so angeordnet sind, dass sie in der Halteposition des Implantats (12) in ergänzende Befestigungsmittel eingreifen, die in einem durch die Abschnitte (9, 10, 30a, 30b) gebildeten Aufnahmeraum (16) angeordnet sind, wobei besagte Mittel eine Gleitverbindung bilden.

2. Vorrichtung (1) nach Anspruch 1, **dadurch gekennzeichnet, dass** die Gleitverbindung so konfiguriert ist, dass eine geradlinige Translationsbewegung des Arretierelements (4) im Verhältnis zum Greifinstrument (3) möglich ist.

3. Vorrichtung (1) nach Anspruch 1 oder Anspruch 2, **dadurch gekennzeichnet, dass** die Gleitverbindung so gestaltet wird, dass eine kreisförmige Translationsbewegung des Arretierelements (4) im Verhältnis zum Greifinstrument (3) möglich ist.

4. Vorrichtung (1) nach einem der Ansprüche 1 bis 3, **dadurch gekennzeichnet, dass** die Abschnitte (9, 10, 30a, 30b) jeweils ein proximales Ende aufweisen, das so angeordnet ist, dass es in das Implantat (2, 20, 200) eingreift.

5. Vorrichtung (1) nach Anspruch 4, **dadurch gekennzeichnet, dass** das Implantat (2, 20, 200) an den proximalen Enden der Abschnitte (9, 10, 30a, 30b) vormontiert ist.

6. Vorrichtung (1) nach einem der Ansprüche 1 bis 5, **dadurch gekennzeichnet, dass** die Befestigungsmittel (12) des Arretierelements (4) schwalbenschwanzförmige Vorsprünge aufweisen, die in die ergänzt geformten Rillen (130) in der Aufnahme (16) eingeschoben werden können und umgekehrt.

7. Vorrichtung (1) nach einem der Ansprüche 1 bis 6, **dadurch gekennzeichnet, dass** die Abschnitte durch zwei Halbschalen (30a, 30b) geformt werden, die durch ein kraftschlüssiges Teil (44) zusammengefügt werden.

8. Vorrichtung (1) nach Anspruch 7, **dadurch gekennzeichnet, dass** das Arretierelement (4) und das kraftschlüssige Teil (44) aus einem einzigen Teil bestehen.

9. Vorrichtung (1) nach Anspruch 7 oder Anspruch 8, **dadurch gekennzeichnet, dass** die beiden Halbschalen (30a, 30b) durch einen am distalen Ende besagter Halbschalen befindlichen Haltering (70) miteinander verbunden sind.

10. Vorrichtung (1) nach einem der Ansprüche 1 bis 9, **dadurch gekennzeichnet, dass** sie eine Zielvorrichtung (50, 80) aufweist, die mindestens eine Öffnung (52, 81) enthält, die das Zielen und Einsetzen eines Befestigungselements in Verbindung mit dem Implantat ermöglicht, nachdem dieses eingesetzt wurde.

11. Vorrichtung (1) nach Anspruch 10, **dadurch gekennzeichnet, dass** die Zielvorrichtung (50, 80) vom Arretierelement (4) gehalten wird.

12. Vorrichtung (1) nach Anspruch 10, **dadurch gekennzeichnet, dass** die Zielvorrichtung (50, 80) vom Greifinstrument (3) gehalten wird.

13. Vorrichtung (1) nach einem der Ansprüche 1 bis 12, **dadurch gekennzeichnet, dass** sie mindestens ein zweites Implantat (90, 120) umfasst, das auf dem Arretierelement (4) vormontiert ist.

14. Vorrichtung (1) nach einem der Ansprüche 11 bis 13, **dadurch gekennzeichnet, dass** das zweite Implantat (90, 120) von der Zielvorrichtung (50, 80) gehalten wird.

15. Vorrichtung (1) nach einem der Ansprüche 1 bis 14, **dadurch gekennzeichnet, dass** das Greifinstrument (3) eine Befestigungsstange (30) ist.

16. Vorrichtung (1) nach einem der Ansprüche 1 bis 15, **dadurch gekennzeichnet, dass** das Greifinstrument (3), wenn das Arretierelement (4) in seinem Innern liegt, ein Führungsrohr bildet.

17. Vorrichtung (1) nach einem der Ansprüche 1 bis 16, **dadurch gekennzeichnet, dass** die Vorrichtung (1) aus Verbundmaterial, Polymer, Eisen- oder Nichteisenlegierung oder einer Kombination dieser Materialien gebildet wird.

18. Vorrichtung (1) nach einem der Ansprüche 1 bis 17, **dadurch gekennzeichnet dass** die Vorrichtung (1) zur einmaligen Benutzung bestimmt ist.

19. Vorrichtung (1) nach einem der Ansprüche 1 bis 18, **dadurch gekennzeichnet, dass** das Implantat (2, 20, 200) ein Oberschenkelimplantat (20) ist.

20. Vorrichtung (1) nach einem der Ansprüche 1 bis 18, **dadurch gekennzeichnet, dass** das Implantat (2, 20, 200) ein Wirbelsäulenimplantat (2, 200) vom Typ Zwischenwirbelkäfig oder Wirbelsäulenosteosyntheseplatte ist.

## Claims

1. A device (1) for positioning an implant (2, 20, 200) comprising:
- an instrument (3) for gripping the implant (2, 20, 200) comprising two portions (9, 10, 30a, 30b) capable of assuming a position for supporting the implant (2, 20, 200) and an open position enabling the implant (2, 20, 200) to be gripped, or released, once same has been implanted, and
- an element (4) for locking the portions (9, 10, 30a, 30b) in the implant supporting position, with the locking element comprising hooking means (13, 130) so designed as to engage with complementary hooking means (12) provided in a receiving cavity (16) formed by the portions (9, 10, 30a, 30b) in the implant supporting position, with said means forming a sliding connection.

2. A device (1) according to claim 1, **characterized in that** the sliding connection is so configured as to enable a rectilinear translation motion of the locking element (4) relative to the gripping instrument (3).

3. A device (1) according to claim 1 or claim 2, **characterized in that** the sliding connection is so configured as to enable a circular translation motion of the locking element (4) relative to the gripping instrument (3).

4. A device (1) according to any one of claims 1 to 3, **characterized in that** the portions (9, 10, 30a, 30b) each have a proximal end so arranged as to engage the implant (2, 20, 200).

5. A device (1) according to claim 4, **characterized in that** the implant (2, 20, 200) is pre-mounted on the proximal ends of the portions (9, 10, 30a, 30b).

6. A device according to any one of claims 1 to 5, **characterized in that** the means for hooking (12) the locking element (4) comprises dovetail-shaped projections adapted to be inserted into grooves (130) having a complementary shape provided in the cavity (16) and vice versa.

7. A device (1) according to any one of claims 1 to 6, **characterized in that** the portions consist of two half-shells (30a, 30b) joined together by means of a connecting element (44).

8. A device (1) according to claim 7, **characterized in that** the locking element (4) and the connecting element (44) are formed integrally.

9. A device (1) according to claim 7 or to claim 8, **characterized in that** the two half-shells (30a, 30b) are joined together by means of a retaining ring (70) positioned at the distal end of said half-shells.

10. A device (1) according to any one of claims 1 to 9, **characterized in that** it comprises a sighting device (50, 80) comprising at least one hole (52, 81) enabling sighting and the positioning of a fastening element associated with the implant once the latter has been positioned.

11. A device (1) according to claim 10, **characterized in that** the sighting device (50, 80) is carried by the locking element (4).

12. A device (1) according to claim 10, **characterized in that** the sighting device (50, 80) is carried by the gripping instrument (3).

13. A device (1) according to any one of claims 1 to 12, **characterized in that** it comprises at least one second implant (90, 120) pre-mounted on the locking element (4).

14. A device (1) according to any one of claims 11 to 13, **characterized in that** the second implant (90, 120) is carried by the sighting device (50, 80).

15. A device (1) according to any one of claims 1 to 14, **characterized in that** the gripping instrument (3) is a mounting tube (30).

16. A device (1) according to any one of claims 1 to 15, wherein the gripping instrument (3), when the locking element (4) is positioned within same, forms a guide tube.

17. A device (1) according to any one of claims 1 to 16, **characterized in that** the device (1) is made of a composite material, a polymer, a ferrous or nonferrous alloy or still a combination of such various materials.

18. A device (1) according to any one of claims 1 to 17, **characterized in that** the device (1) is disposable.

19. A device (1) according to any one of claims 1 to 18, **characterized in that** the device (2, 20, 200) is a femoral implant (20).

20. A device (1) according to any one of claims 1 to 18, **characterized in that** the implant (2, 20, 200) is a spinal implant (2, 200) of the intersomatic cage or a spinal osteosynthesis plate type.
